(19) Europäisches Patentamt / European Patent Office / Office européen des brevets

(11) **EP 2 540 464 A1**

(12) **EUROPEAN PATENT APPLICATION**
published in accordance with Art. 153(4) EPC

(43) Date of publication:
**02.01.2013 Bulletin 2013/01**

(21) Application number: **11747226.6**

(22) Date of filing: **16.02.2011**

(51) Int Cl.:
**B26F 1/38** (2006.01)    **A61F 13/15** (2006.01)
**A61F 13/472** (2006.01)   **A61F 13/49** (2006.01)
**B26F 1/44** (2006.01)     **B65H 35/00** (2006.01)

(86) International application number:
**PCT/JP2011/053252**

(87) International publication number:
**WO 2011/105262 (01.09.2011 Gazette 2011/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.02.2010  JP 2010037846**

(71) Applicant: **Uni-Charm Corporation
Ehime 7990111 (JP)**

(72) Inventor: **NAKANO, Takumi
Kanonji-shi
Kagawa 769-1602 (JP)**

(74) Representative: **Peter, Julian et al
Staeger & Sperling
Partnerschaftsgesellschaft
Sonnenstrasse 19
80331 München (DE)**

(54) **CUTTER DEVICE**

(57)    A cutter apparatus that cuts a workpiece of an absorbent article to be transported in a transport direction including a cutter roller that is provided with a cutter blade that cuts the workpiece, the cutter blade projecting from an outer circumferential face of the cutter roller; an anvil roller that receives the cutter blade with an outer circumferential face thereof disposed so as to oppose the outer circumferential face of the cutter roller; an annular protruding section that is provided to an outer circumferential face of one roller out of the cutter roller and the anvil roller, and that comes into contact with an other of the rollers; a first motor that drives the cutter roller; a second motor that drives the anvil roller; a first motor control unit that controls the first motor by position control or speed control; and a second motor control unit that controls the second motor, wherein the anvil roller rotates to follow the cutter roller by a torque transmitted from the cutter roller via the annular protruding section, and a supplementary torque is imparted from the second motor to the anvil roller, based on a control by the second motor control unit, so as to assist a rotation of the anvil roller following the cutter roller.

FIG. 4

EP 2 540 464 A1

**Description**

[Technical Field]

The present invention relates to a cutter apparatus for cutting workpieces of absorbent articles such as disposable diapers and the like.

[Background Art]

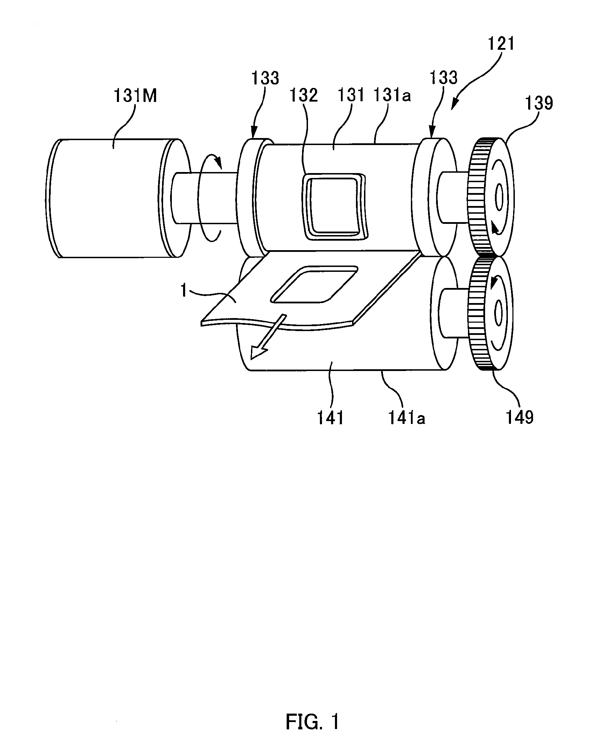

Conventionally, workpieces 1 such as nonwoven fabric are punched out, slits made and cut into units of products and the like along production lines for absorbent articles such as disposable diapers when workpieces 1 are transported in the transport direction, as in the perspective view shown in Fig. 1. In other words, workpieces 1 are cut. And this is performed by the cutter apparatus 121.

The cutter apparatus 121 has a cutter roller 131 with a cutter blade 132 protruding from the outer circumferential face 131a, thereof and a smooth anvil roller 141 that receives the cutter blade 132. And the cutter roller 131 rotates by being driven by the motor 131M, and the anvil roller 141 is applied a rotation torque by the cutter roller 131 via the bearer sections 133, 133 to rotate by following the cutter roller 131. Specifically, a pair of protruding sections 133, 133 referred to as bearer sections are provided in an annular form to the outer circumferential face 131a of the cutter roller 131 in the circumferential direction along its entire circumference. And these annular protruding sections 133, 133 are pressed against the outer circumferential face 141a of the anvil roller 141 into contact and a rotation torque is transmitted from the cutter roller 131 to the anvil roller 141 for the anvil roller 141 to rotate therewith through the frictional force caused by the pressing.

Here, when the moment of inertia of the anvil roller 141 is large, the anvil roller 141 cannot follow the rotation of the cutter roller 131 due to the bearer sections 133, 133 lacking static frictional force when accelerating/decelerating. In other words, the outer circumferential face 141a of the anvil roller 141 would relatively slip in relation to the bearer sections 133 and the cutter blade 132, and thereby abrasion is accelerated at the bearer sections 133 and the cutter blade 132 as well as the outer circumferential face 141a of the anvil roller 141 that abuts thereto.

Therefore, in addition to the bearer sections 133, gears 139, 149 that engage with each other are respectively provided to the shaft portion of the cutter roller 131 and the shaft portion of the anvil roller 141 as shown in Fig. 1. In this way, rotation torque is also transmitted from the cutter roller 131 to the anvil roller 141 (PTL 1).

[Citation List]

[Patent Literature]

[PTL 1]

Japanese Patent Application Laid-open Publication No. 11-188700

[Summary of Invention]

[Technical Problem]

However, even in the latter case, there is fear that relative slipping would occur between the cutter roller 131 and the anvil roller 141 due to a backlash between the gears 139, 149.

The present invention has been made in view of the conventional problems such as those mentioned above, and an object thereof is to provide a cutter apparatus capable of achieving high rotation following performance when the anvil roller is rotated to follow the rotation of the cutter roller using the bearer portions.

[Solution to Problem]

In order to solve the above-described problem, a principal aspect of the invention is, a cutter apparatus that cuts a workpiece of an absorbent article to be transported in a transport direction including;
a cutter roller that is provided with a cutter blade that cuts the workpiece, the cutter blade projecting from an outer circumferential face of the cutter roller;
an anvil roller that receives the cutter blade with an outer circumferential face thereof disposed so as to oppose the outer circumferential face of the cutter roller;

an annular protruding section that is provided to an outer circumferential face of one roller out of the cutter roller and the anvil roller, and that comes into contact with an other of the rollers;

a first motor that drives the cutter roller;

a second motor that drives the anvil roller;

a first motor control unit that controls the first motor by position control or speed control; and

a second motor control unit that controls the second motor; wherein the anvil roller rotates to follow the cutter roller by a torque transmitted from the cutter roller via the annular protruding section, and

a supplementary torque is imparted from the second motor to the anvil roller, based on a control by the second motor control unit, so as to assist a rotation of the anvil roller following the cutter roller.

Features of the invention other than the above will become clear from the description of the present specification and the drawings attached.

[Advantageous Effects of Invention]

According to the present invention, it is possible to achieve high rotation following performance when the anvil roller is rotated to follow the rotation of the cutter roller using the bearer portions.

[Brief Description of Drawings]

[Fig. 1] Fig. 1 is a schematic perspective diagram of a conventional cutter apparatus 121.
[Fig. 2] Fig. 2 is a schematic perspective diagram of a cutter apparatus 21 according to the present embodiment.
[Fig. 3A] Fig. 3A is a vertical sectional view cut along the center of the cutter apparatus 21.
[Fig. 3B] Fig. 3B is a sectional view taken along line B-B of Fig. 3A.
[Fig. 4] Fig. 4 is a schematic configuration diagram of the controller 71.
[Fig. 5] Fig. 5 is a chart showing the driving current I of the first motor 31M and the second motor 41M.
[Fig. 6] Fig. 6 is a flow diagram showing how the cutter apparatus 21 is controlled from activation to normal operation.
[Fig. 7A] Fig. 7A is a vertical sectional view cut along the center of the cutter apparatus 21.
[Fig. 7B] Fig. 7B is a sectional view taken along line B-B of Fig. 3A.
[Fig. 8] Fig. 8 is a schematic configuration diagram of the controller 71 according to another embodiment.

[Mode for Carrying Out the Invention]

At least the following matters will be made clear from the description of the present specification with reference to the accompanying drawings.

A cutter apparatus that cuts a workpiece of an absorbent article to be transported in a transport direction according to the present invention includes a cutter roller that is provided with a cutter blade that cuts the workpiece, the cutter blade projecting from an outer circumferential face of the cutter roller;

an anvil roller that receives the cutter blade with an outer circumferential face thereof disposed so as to oppose the outer circumferential face of the cutter roller;

an annular protruding section that is provided to an outer circumferential face of one roller out of the cutter roller and the anvil roller, and that comes into contact with an other of the rollers;

a first motor that drives the cutter roller;

a second motor that drives the anvil roller;

a first motor control unit that controls the first motor by position control or speed control; and

a second motor control unit that controls the second motor; wherein the anvil roller rotates to follow the cutter roller by a torque transmitted from the cutter roller via the annular protruding section, and

a supplementary torque is imparted from the second motor to the anvil roller, based on a control by the second motor control unit, so as to assist a rotation of the anvil roller following the cutter roller.

According to such a cutter apparatus, supplementary torque is applied from the second motor to the anvil roller and this supplementary torque acts on the anvil roller so to assist the rotation of the anvil roller following the cutter roller. Therefore, the anvil roller is capable of following the rotation of the cutter roller with a high rotation following performance. As a result, relative slipping between the cutter roller and the anvil roller can be restrained enabling to reduce wearing due to the relative slipping.

It is preferable that in the cutter apparatus, the second motor control unit controls the second motor based on a signal indicating a driving torque of the first motor.

According to such a cutter apparatus, the second motor outputs a supplementary torque to the anvil roller based on a signal indicating the driving torque of the first motor. Therefore supplementary torque can be varied by following the rotation of the cutter roller with high rotation following performance. In this way, the anvil roller is capable of swiftly following the cutter roller even when the rotation of the cutter roller accelerates/decelerates.

It is preferable that in the cutter apparatus, the supplementary torque of the second motor is changed to increase or decrease in conjunction with a signal indicating the driving torque of the first motor.

According to such a cutter apparatus, the supplementary torque varies in conjunction with the rotation of the cutter roller. Therefore, the rotation of the anvil roller can be made to follow the rotation of the cutter roller with much higher rotation following performance based on the supplementary torque.

It is preferable that in the cutter apparatus, the first motor generates as the driving torque, a torque of a value corresponding to a value of the driving current to be supplied,

the second motor generates as the supplementary torque, a torque of a value corresponding to a value of the driving current to be supplied,

a signal indicating the driving torque is the driving current of the first motor, and

a driving current supplied to the second motor is changed in proportion to a value of the driving current of the first motor.

According to such a cutter apparatus, the rotation of the anvil roller can be made to follow the rotation of the cutter roller with much higher rotation following performance.

It is preferable that in the cutter apparatus, the second motor control unit changes a driving torque of the second motor in conjunction with a driving torque of the first motor.

According to such a cutter apparatus, the rotation of the anvil roller can be made to follow the rotation of the cutter roller with much higher rotation following performance.

It is preferable that in the cutter apparatus, the first motor control unit and the second motor control unit control the first motor and the second motor, respectively, based on position control or speed control at a start of rotation of the cutter roller and the anvil roller, thereby driving the cutter roller and the anvil roller to rotate such that a deviation between a circumferential speed of the cutter roller and a circumferential speed of the anvil roller falls within a predetermined range, the annular protruding section of one of the rollers is pressed into contact with the outer circumferential face of the other of the rollers concurrent or almost concurrent with the start of the rotation, thereafter,

the second motor control unit switches the control of the second motor from the position control to an interlocking torque control, and

the second motor control unit, under the interlocking torque control, controls a driving torque of the second motor based on a signal indicating a driving torque of the first motor.

According to such a cutter apparatus, the second motor can be operated smoothly based on interlocking torque control while restraining relative slipping between the cutter roller and the anvil roller.

Further, when starting rotation, the tolerance between the rotational speed of the cutter roller and the rotational speed of the anvil roller can be reduced quickly, since the first motor and the second motor are controlled by position control or by speed control.

=== Present Embodiment ===

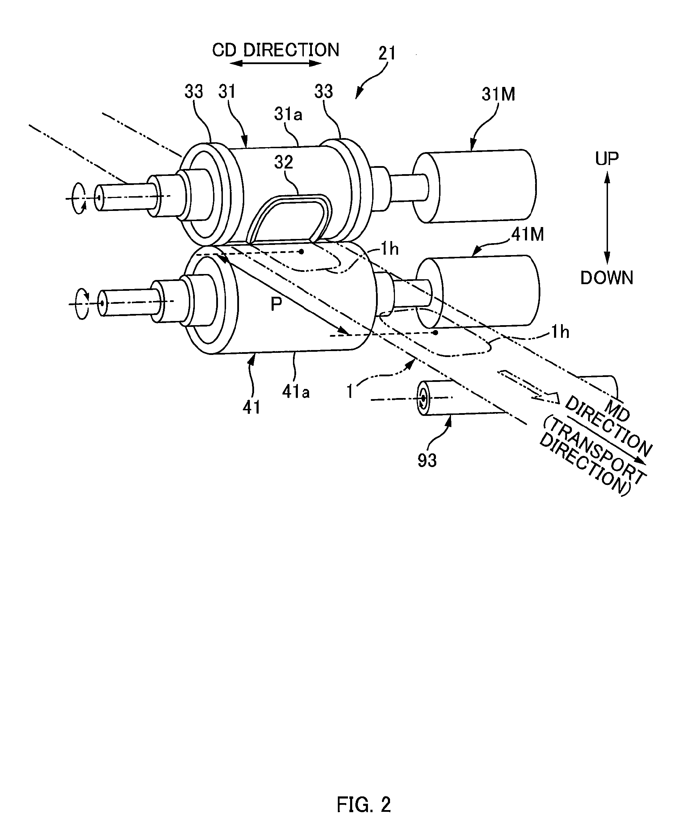

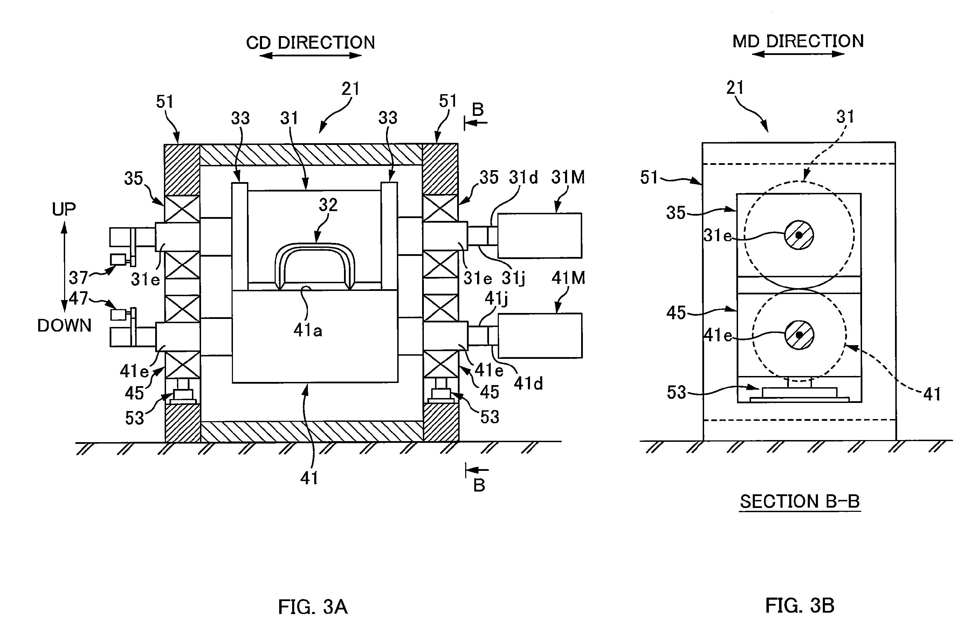

Fig. 2 through Fig. 3B are explanatory diagrams of the cutter apparatus 21 according to the present embodiment. Fig. 2 is a schematic perspective diagram of the cutter apparatus 21, Fig. 3A is vertical sectional view cut along the center of the cutter apparatus 21 and Fig. 3B is a sectional view taken along line B-B of Fig. 3A.

The cutter apparatus 21 is used in a production line for absorbent articles such as disposable diapers. And in this example, the cutter apparatus 21 is made to punch out leg opening sections 1h of the diapers at a product pitch of the diapers from the semi-processed product 1 (corresponding to a "workpiece") in a form of a continuous sheet-like member made of nonwoven fabric and the like being continuously transported in the transport direction. Note that the semi-processed product 1 is not limited to a continuous sheet-like member. That is to say, the semi-processed product 1 may be in the form of separated units each corresponding to the diaper product, and the separated units may be individually transported. In this case, as a transport mechanism for transporting the semi-processed products 1, such as a suction conveyor (belt conveyor with load bearing surfaces having an adsorption function) is used instead of a transport roller 93.

In the following description, the direction in which such a semi-processed product 1 is transported is referred to as also the "MD direction", and, among the directions orthogonal to the MD direction, the direction not in the thickness direction of the semi-processed product 1 (the width direction of the sheet-like member when the semi-processed product 1 is a continuous sheet-like member) is referred to as also the "CD direction".

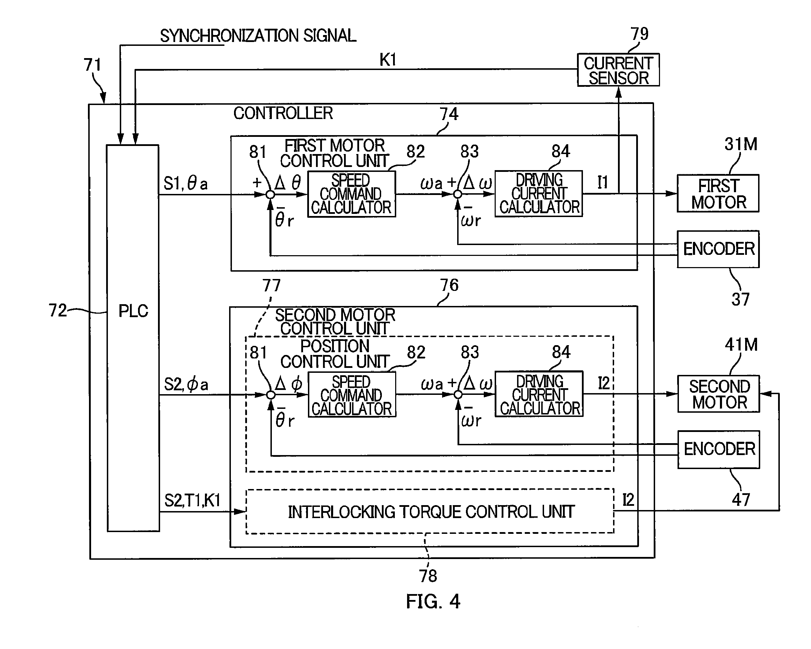

The cutter apparatus 21 includes a cutter roller 31 rotatably supported about a rotational shaft parallel to the CD direction, an anvil roller 41 rotatably supported about a rotational shaft parallel to the CD direction, a first motor 31M to drive the cutter roller 31 to rotate, a second motor 41M to drive the anvil roller 41 to rotate, and a controller 71 to control the first motor 31M and the second motor 41M (see Fig. 4).

And when the semi-processed product 1 is conveyed in the MD direction being the transport direction between the cutter roller 31 and the anvil roller 41 both driven to rotate, the cutter blade 32 on the outer circumferential face 31a of the cutter roller 31 and the outer circumferential face 41a of the anvil roller 41 sandwiches the semi-processed product 1 to cut out the leg opening section 1h from the semi-processed product 1.

The cutter roller 31 has a cutter blade 32 and bearer sections 33 on its outer circumferential face 31a. The cutter blade 32 is provided so to protrude out from the outer circumferential face 31a in the form in which the semi-processed product 1 is to be cut. Additionally, the turning radius at the tip of the cutter blade 32 is set to, for example, a value approximately equal to the value obtained by dividing the product pitch P of the diapers by $2\pi$ (twice the value of the circular constant). Hereby, while the cutter roller 31 rotates once, the semi-processed product 1 is conveyed in the MD direction by a distance of P corresponding to the length of a piece of the product.

Meanwhile, the bearer sections 33, 33 are portions with large diameters (corresponding to the ring-shaped projecting portions) provided to each end portions of the cutter roller 31 in the CD direction. And the bearer sections 33, 33 are pressed against the outer circumferential face 41a of the anvil roller 41 into contact therewith when the cutter apparatus 21 is under normal operation to impart rotational force to the anvil roller 41 through static frictional force caused by the pressing. In other words, even though the anvil roller 41 has a driving motor it being the second motor 41M exclusive for itself, this second motor 41M is strictly for the purpose of assisting the rotational movement of the anvil roller 41 and is basically a slave roller that rotates to follow the rotation of the cutter roller 31. Further, it can be said that the cutter roller 31 and the anvil roller 41 are in a master-slave relation with regard to rotational movement. Explanation with regard to this will be given later.

The radius at the circumferential face (the surface pressed against the anvil roller 41 into contact) of the bearer section 33 is set to, for example, a value same as the turning radius at the tip of the cutter blade 32. However, the radiuses are not limited to such values as long as the cutting ability of the cutter blade 32 and the transmissibility of the rotation torque transmitted via the bearer sections 33 to the anvil roller 41 are not disturbed, in other words, one of the radiuses may be larger than the other.

The anvil roller 41 is for example, a plane roll with its outer circumferential face 41a on the same plane in the CD direction. That is, the radius of the anvil roller 41 is uniform along the entire width in the CD direction in this example. However, the roller profile (the outer circumferential profile) at the outer circumferential face 41a of the anvil roller 41 may be modified from a planer one as long as the cutting ability of the cutter blade 32 and the transmissibility of the rotation torque transmitted through the bearer sections 33 to the anvil roller 41 are not disturbed.

The above-mentioned contact force causing the bearer sections 33, 33 to come into contact with the anvil roller 41 is imparted by a contact force imparting mechanism such as a hydraulic cylinder 53. Specifically, with reference to Figs. 3A and 3B, the cutter apparatus 21 has housings 51, 51 on both sides in the CD direction to support the cutter roller 31 and the anvil roller 41 as shown in Fig. 3A. The housings 51 are frame members 51 in substantially rectangular form as shown in Fig. 3B and the end portions 31e of the cutter roller 31 and the end portions 41e of the anvil roller 41 are placed inside the frame members 51 via bearings 35, 45, respectively. Further, hydraulic cylinders 53, 53 are respectively interposed between the end portions 41e, 41e of the anvil roller 41 and the housings 51, 51 corresponding thereto, and the anvil roller 41 is supported via the bearings 45, 45 to be vertically elevatable by an operation of these hydraulic cylinders 53, 53. Accordingly, the contact force can be varied according to one's choice by adjusting the oil pressure value of operating oil that operates the hydraulic cylinders 53, 53. Note that, the hydraulic cylinders 53, 53 may be interposed between the end sections 31e, 31e of the cutter roller 31 and the housings 51, 51 corresponding thereto. Further, an air cylinder or a feed screw mechanism may be used as a contact force imparting mechanism in place of the hydraulic cylinder 53.

The first motor 31M is a direct-current motor that rotates the rotational driveshaft 31d by a torque of a value according to the value of the driving current I1 supplied. And as shown in Fig. 3A, this rotational driveshaft 31d is connected to the end portion 31e of the cutter roller 31 via an appropriate shaft coupling 31j such as a flexible coupling. In this way, a driving torque is imparted to the cutter roller 31 for the cutter roller 31 to drivingly rotate about the rotational shaft. Note that the cutter roller 31 is provided with an encoder 37 which detects at real time the rotation angle (rotational position) and the angular velocity of the cutter roller 31. For example, the encoder 37 sequentially outputs in proportion to the amount of rotation of the cutter roller 31, a predetermined continuous set of values (for example, 8192 digital values from zero to 8191) while the cutter roller 31 makes a single rotation. Further, the encoder also sequentially outputs the temporal differentiation values of the above values. Note that in the following, description is given on the premise that the rotation angle and the angular velocity are output as the above values and the temporal differentiation values.

The second motor 41M is also a direct-current motor that rotates the rotational driveshaft 41d by a torque of a value according to the value of the driving current I2 supplied. And this rotational driveshaft 41d is also connected to the end portion 41e of the anvil roller 41 via an appropriate shaft coupling 41j. In this way, a driving torque is imparted to the anvil roller 41 for the anvil roller 41 to drivingly rotate about the rotational shaft. Further, the anvil roller 41 relating to the second motor 41M is also provided with an encoder 47 similar to the above-mentioned cutter roller 31 relating to the first motor 31M.

Fig. 4 is a schematic configuration diagram of the controller 71.

The controller 71 includes a PLC 72, a first motor control unit 74 that controls the drive of the first motor 31M based on the first control signal S1 sent from the PLC 72, and a second motor control unit 76 that controls the drive of the second motor 41M based on the second control signal S2 sent from the PLC 72.

The PLC 72 (programmable logic controller) includes a processor. And the above described control signals S1, S2 are generated and the like to be output to the first motor control unit 74 and the second motor control unit 76 by a processor executing programs prestored in the PLC 72.

The first motor control unit 74 controls the first motor 31M by so-called position control. In other words, when a signal indicating a command value of the rotation angle (rotational position) $\theta a$ as the first control signal S1 is sent from the PLC 72 to the first motor control unit 74, the first motor control unit 74 outputs a driving current I1 to the first motor 31M such that the deviation $\Delta\theta$ between the command value of rotation angle $\theta a$ and the actual value of the rotation angle $\theta r$ output from the above-mentioned encoder 37 as the actual rotation angle, becomes small.

Specifically, the first motor control unit 74 includes a position comparator 81, a speed command calculator 82, a speed comparator 83, and a driving current calculator 84. The position comparator 81 compares the command value of rotation angle $\theta a$ with the actual value of the rotation angle $\theta r$ to calculate the deviation $\Delta\theta$ (deviation angle $\Delta\theta$) between the two. And this deviation angle $\Delta\theta$ is input into the speed command calculator 82. The speed command calculator 82 calculates the angular velocity (rotational speed) command value $\omega a$ by a predetermined calculation based on the aforementioned deviation angle $\Delta\theta$ and sends this command value $\omega a$ to the speed comparator 83. Then the speed comparator 83 compares this angular velocity command value $\omega a$ with the angular velocity actual value $\omega r$ sent from the encoder 37 to calculate the deviation $\Delta\omega$ (angular velocity deviation $\Delta\omega$) between the two. Then this angular velocity deviation $\Delta\omega$ is sent to the driving current calculator 84. The driving current calculator 84 performs a predetermined calculation based on the aforementioned angular velocity deviation $\Delta\omega$ to obtain the value of the driving current I1 that lowers the angular velocity deviation $\Delta\omega$. The driving current I1 obtained, is supplied to the first motor 31M to drive the first motor 31M.

The second motor control unit 76 is configured to be executable by switching alternatively between position control and interlocking torque control (a control that changes the driving torque of the second motor 41M in response to the movement of the driving torque of the first motor 31M). Further, the position control is selected when activating (i.e., at start of rotation) the cutter apparatus 21, and the interlocking torque control is selected during normal operation (i.e., when punching out leg opening sections 1h of the semi-processed product 1) of the cutter apparatus 21.

When position control is selected, a signal indicating the rotation angle (rotational position) command value $\varphi a$ as the second control signal S2 is sent from PLC 72 to the second motor control unit 76, and the second motor control unit 76 outputs a driving current I2 to the second motor 41M that lowers the deviation $\Delta\varphi$ between the rotation angle command value $\varphi a$ and the rotation angle actual value $\varphi r$ sent from the aforementioned encoder 47 being the actual rotation angle. This position control is performed by the position control unit 77 equipped by the second motor control unit 76 and the basic configuration thereof is approximately the same as that of the aforementioned first motor control unit 74 as shown in Fig. 4, therefore detailed description thereof is omitted.

On the other hand, when the interlocking torque control is selected, the interlocking torque control unit 78 is selected instead of the aforementioned position control unit 77 at the second motor 1 control unit 76. Then a signal indicating the driving torque T1 of the first motor 31M as the second control signal S2 is sent at real time from the PLC 72 to the interlocking torque control unit 78. Specifically, a signal indicating a value K1 of the driving current I1 of the first motor 31M is sent from the PLC 72 at real time. Then the value of the driving current I2 to be supplied to the second motor 41M is sequentially calculated based on this signal at the interlocking torque control unit 78, and this driving current I2 is supplied to drive the second motor 41M.

Here, the value of the driving current I2 to be supplied to this second motor 41M is calculated in the following way. First, value K1 of the driving current I1 of the first motor 31M is sequentially measured with the current sensor 79. Then the value K1 of the measured driving current I1 is multiplied by a constant A. In other words, the following formula (1) is used.

$$\text{value of the driving current I2 to be supplied} = A \times K1 \qquad (1)$$

Note that, the constant A is a fixed value determined in accordance with terms and conditions such as the moment of inertia of the cutter roller 31 and the anvil roller 41, the frictional resistance of the bearings 35, 45 and the acceleration and deceleration pattern of the assumed transportation speed of the semi-processed product during normal operation.

For example, the moment of inertia and the frictional resistance of the bearings 35, 45 between the cutter roller 31 and the anvil roller 41 are approximately the same and this constant A is selected from a value greater than zero and smaller than one when the motors 31M and 41M have the same specification. Specifically, this constant A is set to a value greater than zero and less than 0.3, or in some cases to a value equal to or greater than 0.3 and less than 0.5, furthermore in some cases to a value equal to or greater than 0.5 and less than one. Note that, the range among the three ranges above from which the value of the constant A is selected is determined according to the pattern of acceleration/deceleration or the magnitude of the above-mentioned contact force. For example, the constant A is selected from the first range in a case the acceleration/deceleration pattern is relatively gentle and a large static frictional force at the bearer sections 33 can be secured by adjusting the contact force, whereas the constant A is selected from the third range in a case the acceleration/deceleration pattern is relatively steep and a large static frictional force at the bearer sections 33 cannot be secured by adjusting the contact force, and the constant A is selected from the second range in a case under a condition between the above two.

However, the constant A may be selected by trial and error using an experimental technique. In other words, the constant A is set to a fixed value, the cutter apparatus 21 is operated in an acceleration/deceleration pattern and under a contact force of an actual operation and after the operation, the surface conditions of the bearer sections 33 and the surface condition of the section of the anvil roller 41 that comes into contact with the bearer sections 33 are examined to check whether relative slipping has occurred between the two. Then a condition at which relative slipping does not occur, that is, a condition where the pressing between the bearer sections 33 and the anvil roller 41that, generally maintains the static frictional state may be figured out by repeating this work while varying the value of constant A among a plurality of levels. Note that, the contact force may further be varied among a plurality of levels when there is difficulty in figuring out such condition. That is, since the static frictional force at the bearer sections 33 grows when increasing the contact force, a condition that maintains the static frictional state at the bearer sections 33 during normal operation will be found easier.

Then, when the constant A is set in the above manner, the rotation of the anvil roller 41 during normal operation becomes the slave when the rotation of the cutter roller 31 driven to rotate is the master. That is, basically, the anvil roller 41 becomes the slave roller that rotates to follow the cutter roller 31 by a rotation torque implemented by the cutter roller 31 via the bearers 33. Therefore, the second motor 41M is strictly for the purpose of imparting a supplementary torque to the anvil roller 41 to assist the anvil roller 41 to follow the rotation of the cutter roller 31. In this way, the anvil roller 41 can follow the rotation of the cutter roller with high following performance based on the imparted supplementary torque. As a result, the cutter roller 31 and the anvil roller 41 rotate at approximately the same speed at portions such as the bearer sections 33 where the two come into contact thereby being able to restrain relative slipping between the two and effectively reduce abrasion due to this relative slipping.

Additionally, even in a case the rotation of the cutter roller 31 involves acceleration/deceleration, as can be understood from the above formula (1), the anvil roller 41 can swiftly follow the rotation of the cutter roller 31 since the driving torque of the second motor 41M increases/decreases in conjunction with the increase/decrease of the driving torque of the first motor 31M.

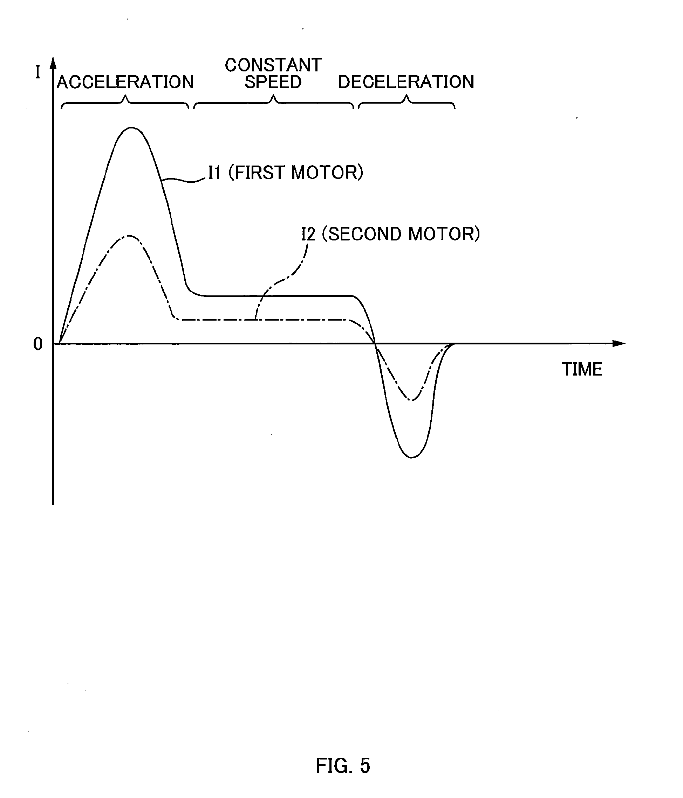

For example, the chart of the driving current I of the first motor 31M and the second motor 41M shown in Fig. 5 indicates the driving current I2 of the second motor 41M taking a positive value during acceleration similar to the driving current I1 of the first motor 31M, whereas the driving current I2 of the second motor 41M takes a negative value along with the driving current I1 of the first motor 31M during deceleration. Therefore, the anvil roller 41 swiftly follows the rotation of the cutter roller 31 not only when moving at constant speed but also during acceleration and deceleration.

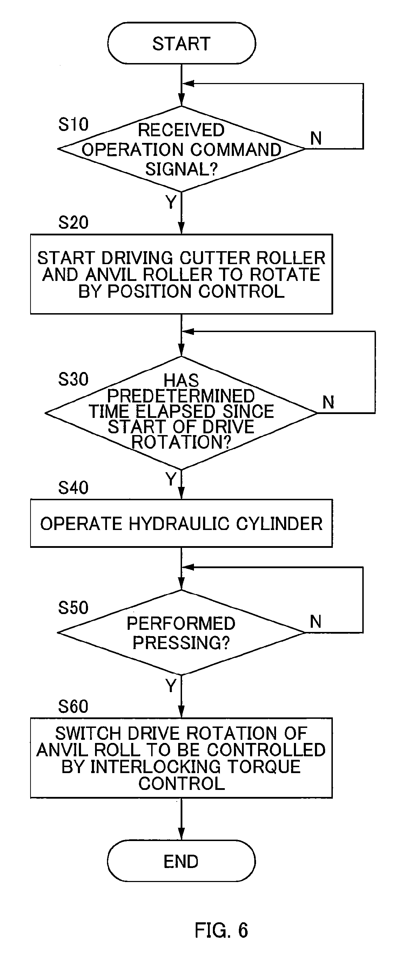

Fig. 6 is a flow diagram showing how the cutter apparatus 21 is controlled from activation to normal operation.

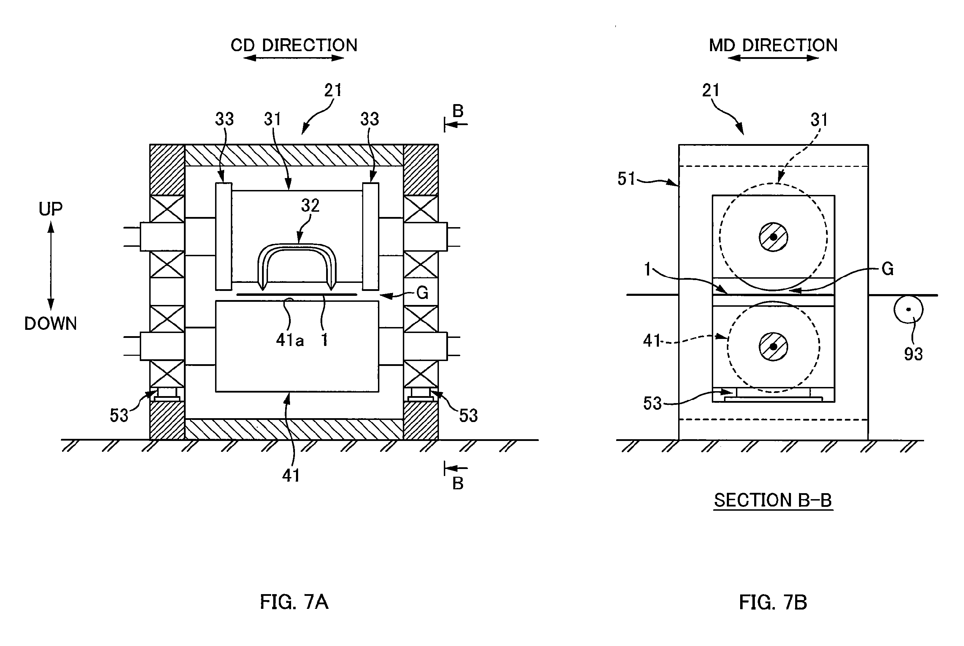

First, when the cutter apparatus 21 is activated, the cutter roller 31 and the anvil roller 41 are stopped from rotating as shown in Figs. 7A and 7B. And the anvil roller 41 is at the lowermost limit thereby forming a gap G between the outer circumferential face 41a of the anvil roller 41 and the cutter blade 32 as well as the bearer sections 33 of the cutter roller 31. Further, this gap G has a continuous sheet-like member 1 as the semi-processed product 1 passing therethrough in the MD direction.

Next, the controller on the main equipment side, not shown, that controls the transport roller 93 starts operating the transport roller 93 when an operator presses the operation start button of the control panel of the production line, and thereby the continuous sheet-like member 1 is transported at a predetermined transportation speed. Further, an operation command signal is sent from the controller on the main equipment side to the PLC 72 of the cutter apparatus 21 (S10). Then the PLC 72 that receives this signal starts to drive the cutter roller 31 and the anvil roller 41 to rotate controlled by position control (S20).

Specifically, first the PLC 72 receives a synchronization signal from the encoder (not shown) of the transport roller 93. This synchronization signal is, similar to the aforementioned encoders 37, 47, a signal having a predetermined continuous set of values (for example, 8192 digital values from zero to 8191) and this synchronization signal has a digital value between zero and 8191 output in proportion to the amount of the semi-processed product 1 transported while the semi-processed product 1 is transported by a distance equal to the product pitch P.

Next, the PLC 72 generates signals of the command values of rotation angles θa, φa of the cutter roller 31 and the anvil roller 41 based on the received synchronization signal and outputs the generated signals to the first motor control unit 74 and the second motor control unit 76, respectively. Then the first and second motor control units 74, 76 respectively control the first motor 31M and the second motor 41M by position control based on the received command values θa, φa to drive the cutter roller 31 and the anvil roller 41 to rotate.

Here, the rolls 31, 41 are respectively position controlled based on the command values of rotation angles θa, φa generated based on the synchronization signal so that after a predetermined time elapses, both the cutter roller 31 and the anvil roller 41 rotate in approximately the same speed as the transportation speed of the semi-processed product 1. In other words, the deviation between the circumferential speed of the cutter roller 31 and the circumferential speed of the anvil roller 41 is within a range such that a problem would not occur when the rollers 31, 41 come into contact with each other.

Therefore, the PLC 72 operates the hydraulic cylinders 53, 53 to press these rollers 31, 41 against each other after a predetermined time elapsed after starting the rollers 31, 41 to be driven to rotate (S30, S40). In other words, the hydraulic pressure value of the hydraulic cylinders 53, 53 are increased from zero to a predetermined level to raise the anvil roller 41 from a state shown in Figs. 7A and 7B to a state shown in Figs. 3A and 3B. In this way, the anvil roller 41 is pressed to come into contact with the cutter roller 31 by a predetermined contact force.

When the PLC 72 detects this pressing based on the changes in hydraulic pressure value and the elapsed time after starting operation of the hydraulic cylinder 53, the PLC 72 switches the second motor control unit 76 responsible for controlling the anvil roller 41 from position control to interlocking torque control (S50, S60). For example, the PLC 72 sends a signal of the measured value K1 of the driving current I1 of the first motor 31M to the second motor control unit 76 instead of a signal indicating the rotation angle command value φa. Thereby, the control unit to be in operation in the second motor control unit 76 is switched from the position control unit 77 to the interlocking torque control unit 78. And thereafter, the anvil roller 41 is imparted a supplementary torque from the second motor 41M based on interlocking torque control. In this way, the cutter apparatus 21 comes under normal operation. That is, the anvil roller 41 is imparted a rotation torque through static frictional force from the bearer sections 33 of the cutter roller 31 while being imparted a supplementary torque from also the second motor 41M, and herewith, the anvil roller 41 rotates to follow the cutter roller 31.

Note that in the above-mentioned example, the cutter roller 31 and the anvil roller 41 were pressed to come into contact after starting to rotate these rollers 31, 41, however, the order in which these are performed may be reversed. In other words, with the cutter roller 31 and the anvil roller 41 in states stopped from rotating, first the anvil roller 41 is raised by the operation of the hydraulic cylinder 53 to be pressed to come into contact with the cutter roller 31 and thereafter, the rotation of the cutter roller 31 and the anvil roller 41 may be started by position control. By the way, the anvil roller 41 is switched from position control to interlocking torque control after starting this rotation.

=== Other Embodiments ===

Hereinabove, description was given of an embodiment of the present invention, however, the present invention is not to limited to such an embodiment and may be modified in the following ways.

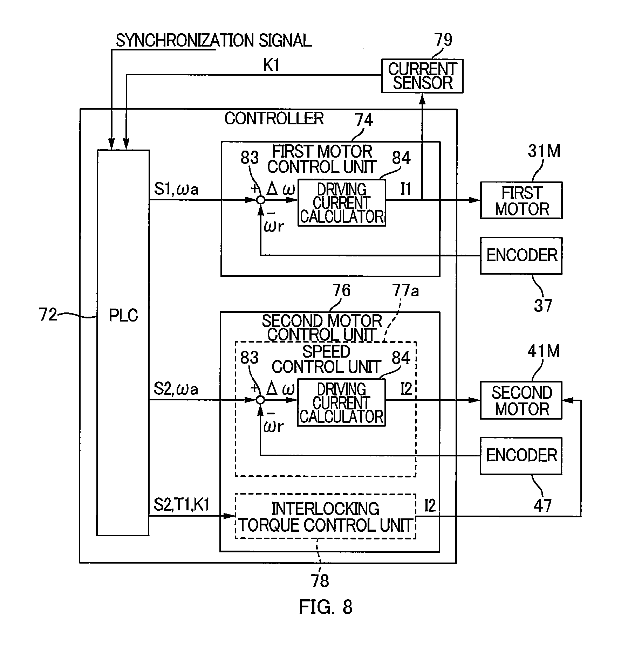

In the aforementioned embodiment, the first motor control unit 74 and the second motor control unit 76 performed position control, however, speed control may be performed instead of position control. That is, as shown in Fig. 8, a signal indicating angular velocity (rotational velocity) command value ωa may be sent as control signals S1, S2 from the PLC 72 to the respective control units 74, 76, and driving currents I1, I2 may be output to the corresponding motors 31M, 41M such that the deviation Δω between this angular velocity command value ωa and the actual value of the angular velocity ωr output from the respective encoders 37, 47 as the actual angular velocity becomes small.

Detailed description will be given with reference to Fig. 8. First, the second motor control unit 76 has a speed control unit 77a instead of a position control unit 77. And both the first motor control unit 74 and the aforementioned speed control unit 77a have a speed comparator 83 and a driving current calculator 84. And the speed comparator 83 compares this angular velocity command value ωa with the actual value of the angular velocity ωr sent from the encoder 37 (47) to calculate the deviation Δω (angular velocity deviation Δω) between the two and sends this angular velocity deviation Δω to the driving current calculator 84. Then a predetermined calculation is performed based on this angular velocity deviation Δω at the driving current calculator 84 to obtain a driving current I1 (I2) that makes this angular velocity deviation Δω small and supplies the obtained driving current I1 (I2) to the motor 31M (41M) in charge.

In the aforementioned embodiment, the bearer sections 33 were provided integral with the cutter roller 31, however, the mechanism is not limited to such as long as rotation torque can be transmitted from the cutter roller 31 to the anvil roller 41, that is, the bearer sections 33 may be provided integral with the anvil roller 41.

In the aforementioned embodiment, a direct current (DC) motor was shown as an example of the first and second motors 31M, 41M, however, the motor type is not limited to such as long as they are electric motors and alternating-current (AC) motors can be used.

In the aforementioned embodiment, disposable diapers worn by a wearer to absorb excretion of the wearer were shown as an example of an absorbent article, however, the absorbent article is not limited to such as long as it absorbs excretion such as urine and menses and, for example, sanitary napkins and pet waste pads that absorb liquid excretion of pets, and the like can be used.

In the aforementioned embodiment, the driving current I2 of the second motor 41M was varied in proportion to value K1 of the driving current I1 of the first motor 31M as interlocking torque control, however, interlocking torque control is not limited to such. For example, with the driving current I2 defined with an appropriate function f (K1) having value K1 of the driving current I1 as a variable, the value of the driving current I2 may be varied based on this function f (K1).

In the aforementioned embodiment, an example was shown where the moment of inertia and the frictional resistance of the bearings of the cutter roller 31 were approximately equal to those of the anvil roller 41, however, they need not be equal. Note that, in such a case, the aforementioned function f (K1) and the constant A is considered to be found easier when the moment of inertia and the frictional resistance of the cutter roller 31 are greater than those of the anvil roller 41. In other words, the anvil roller 41 would be made easier to rotate by following the cutter roller 31.

In the aforementioned embodiment, description was given of an example where the specifications of the first motor 31M and the second motor 41M were the same, however, the specification is not limited to such and the specifications of the two need not be the same.

In the aforementioned embodiment, the driving current I2 of the second motor 41M was varied based on the value K1 of the driving current I1 of the first motor 31M as an example of interlocking torque control, however, the interlocking torque control is not limited to such. For example, with the driving torque of the first motor 31M measured with a torquemeter, the driving current I2 of the second motor 41M may be controlled to vary the value of the driving torque T2 of the second motor 41M in conjunction with the measured value Q1 of the measured torque T1 (for example, refer to the following formula (2)).

$$\texttt{value of driving torque T2 of second motor 41M} = \texttt{A} \times \texttt{Q1} \qquad (2)$$

Note that, in this case, a signal indicating the measured value Q1 of the above-mentioned torque T1 corresponds to the signal indicating the driving torque of the first motor 31M.

[Reference Signs List]

1 semi-processed product (workpiece, continuous sheet-like member), 1h leg opening section, 21 cutter apparatus, 31 cutter roller, 31a outer circumferential face, 31e end portion , 31M first motor, 31d rotational driveshaft, 31j shaft coupling, 32 cutter blade, 33 bearer sections (annular protruding sections, large diameter sections), 37 encoder, 41 anvil roller, 41a outer circumferential face, 41e end portion , 41M second motor, 41d rotational driveshaft, 41j shaft coupling, 47 encoder, 51 housing (frame member), 53 hydraulic cylinder, 71 controller, 74 first motor control unit, 76 second motor control unit, 77 position control unit, 77a speed control unit, 78 interlocking torque control unit, 79 current sensor, 81 position comparator, 82 speed command calculator, 83 speed comparator, 84 driving current calculator, 93 transport roller, G gap

## Claims

1. A cutter apparatus that cuts a workpiece of an absorbent article to be transported in a transport direction comprising;
   a cutter roller that is provided with a cutter blade that cuts the workpiece, the cutter blade projecting from an outer circumferential face of the cutter roller;
   an anvil roller that receives the cutter blade with an outer circumferential face thereof disposed so as to oppose the outer circumferential face of the cutter roller;
   an annular protruding section that is provided to an outer circumferential face of one roller out of the cutter roller and the anvil roller, and that comes into contact with an other of the rollers;
   a first motor that drives the cutter roller;
   a second motor that drives the anvil roller;
   a first motor control unit that controls the first motor by position control or speed control; and
   a second motor control unit that controls the second motor; wherein the anvil roller rotates to follow the cutter roller by a torque transmitted from the cutter roller via the annular protruding section, and
   a supplementary torque is imparted from the second motor to the anvil roller, based on a control by the second motor control unit, so as to assist a rotation of the anvil roller following the cutter roller.

2. The cutter apparatus according to claim 1, wherein
the second motor control unit controls the second motor based on a signal indicating a driving torque of the first motor.

3. The cutter apparatus according to claim 2, wherein
the supplementary torque of the second motor is changed to increase or decrease in conjunction with a signal indicating the driving torque of the first motor.

4. The cutter apparatus according to claim 3, wherein
the first motor generates as the driving torque, a torque of a value corresponding to a value of the driving current to be supplied,
the second motor generates as the supplementary torque, a torque of a value corresponding to a value of the driving current to be supplied,
a signal indicating the driving torque is the driving current of the first motor, and
a driving current supplied to the second motor is changed in proportion to a value of the driving current of the first motor.

5. The cutter apparatus according to one of claims 1 to 4, wherein
the second motor control unit changes a driving torque of the second motor in conjunction with a driving torque of the first motor.

6. The cutter apparatus according to one of claims 1 to 5, wherein
the first motor control unit and the second motor control unit control the first motor and the second motor, respectively, based on position control or speed control at a start of rotation of the cutter roller and the anvil roller, thereby driving the cutter roller and the anvil roller to rotate such that a deviation between a circumferential speed of the cutter roller and a circumferential speed of the anvil roller falls within a predetermined range,
the annular protruding section of one of the rollers is pressed into contact with the outer circumferential face of the other of the rollers concurrent or almost concurrent with the start of the rotation,
thereafter,
the second motor control unit switches the control of the second motor from the position control to an interlocking torque control, and
the second motor control unit, under the interlocking torque control, controls a driving torque of the second motor based on a signal indicating a driving torque of the first motor.

FIG. 1

CD DIRECTION

33 31 33 21 31M

31a

32

1h 41M

UP

P

1h DOWN

41 41a 1

93

MD DIRECTION
(TRANSPORT DIRECTION)

FIG. 2

CD DIRECTION

MD DIRECTION

FIG. 3A

FIG. 3B

SECTION B-B

UP

DOWN

FIG. 4

FIG. 5

FIG. 6

FIG. 7B

FIG. 7A

FIG. 8

## INTERNATIONAL SEARCH REPORT

| | International application No. |
| --- | --- |
| | PCT/JP2011/053252 |

A. CLASSIFICATION OF SUBJECT MATTER
*B26F1/38*(2006.01)i, *A61F13/15*(2006.01)i, *A61F13/472*(2006.01)i, *A61F13/49*
(2006.01)i, *B26F1/44*(2006.01)i, *B65H35/00*(2006.01)i

According to International Patent Classification (IPC) or to both national classification and IPC

B. FIELDS SEARCHED

Minimum documentation searched (classification system followed by classification symbols)
B26F1/38, A61F13/15, A61F13/472, A61F13/49, B26F1/44, B65H35/00

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 2003-94390 A  (O-M Ltd.),<br>03 April 2003 (03.04.2003),<br>paragraphs [0014] to [0036]<br>(Family: none) | 1<br>2-5<br>6 |
| Y | JP 2007-319992 A  (Reliance Electric Ltd.),<br>13 December 2007 (13.12.2007),<br>claims 1 to 3<br>(Family: none) | 2-5 |

☒  Further documents are listed in the continuation of Box C.          ☐  See patent family annex.

| | | |
| --- | --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>    09 May, 2011 (09.05.11) | Date of mailing of the international search report<br>    17 May, 2011 (17.05.11) |
| --- | --- |
| Name and mailing address of the ISA/<br>    Japanese Patent Office | Authorized officer |
| Facsimile No. | Telephone No. |

Form PCT/ISA/210 (second sheet) (July 2009)

EP 2 540 464 A1

**INTERNATIONAL SEARCH REPORT**

International application No.

PCT/JP2011/053252

C (Continuation).   DOCUMENTS CONSIDERED TO BE RELEVANT

| Category* | Citation of document, with indication, where appropriate, of the relevant passages | Relevant to claim No. |
|---|---|---|
| A | CD-ROM of the specification and drawings annexed to the request of Japanese Utility Model Application No. 55384/1992 (Laid-open No. 24900/1994) (Hamada Printing Press Co., Ltd.), 05 April 1994 (05.04.1994), entire text (Family: none) | 1-6 |
| A | JP 56-157993 A  (Toshiba Machine Co., Ltd.), 05 December 1981 (05.12.1981), entire text (Family: none) | 1-6 |

Form PCT/ISA/210 (continuation of second sheet) (July 2009)

20

**EP 2 540 464 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11188700 A